# EUROPÄISCHE PATENTSCHRIFT

(11) **EP 1 786 868 B1**
(45) Veröffentlichungstag und Bekanntmachung des Hinweises auf die Patenterteilung: **27.01.2010**
(21) Anmeldenummer: 05785760.9
(22) Anmeldetag: 10.08.2005
(51) Int. Cl.: C09C 1/00

(54) **PERLGLANZPIGMENTE**
NACREOUS PIGMENTS
PIGMENTS NACRES

(30) Priorität: 13.08.2004 DE 102004039554
(43) Veröffentlichungstag der Anmeldung: 23.05.2007
(73) Patentinhaber: Merck Patent GmbH, 64293 Darmstadt (DE)
(72) Erfinder: MELSON, Sabine, 64367 Mühltal (DE); ENTENMANN, Marc, 70734 Fellbach (DE); JEKEL, Marita, 64287 Darmstadt (DE); MATHIAS, Marcus, 64579 Gernsheim (DE)
(86) Internationale Anmeldenummer: PCT/EP2005/008658
(87) Internationale Veröffentlichungsnummer: WO 2006/018196

(56) Entgegenhaltungen:
- EP-A- 0 644 242
- WO-A-03/006558
- WO-A-2004/055119

## Beschreibung

Die vorliegende Erfindung betrifft Perlglanzpigmente auf der Basis von mehrfach beschichteten Glasplättchen sowie deren Verwendung, u.a. in Farben, Lacken, Druckfarben, Kunststoffen und in kosmetischen Formulierungen. Die erfindungsgemäßen Pigmente sind insbesondere geeignet zur Pigmentierung von Kunststoffen, da sie eine erhöhte chemische und mechanische Stabilität aufweisen.

Silberweißpigmente werden als Glanz- oder Effektpigmente in vielen Bereichen der Technik eingesetzt, insbesondere in der dekorativen Beschichtung, im Kunststoff, in Farben, Lacken, Druckfarben sowie in kosmetischen Formulierungen.

Kunststoffe kommen im Außen- und Innenbereich zur Anwendung und enthalten zur Verbesserung der Eigenschaften in der Regel eine Reihe von Zusatzstoffen, wie z.B. Weichmacher, Füllstoffe, Stabilisatoren und Alterungsschutzmittel, Gleit- und Trennmittel, Antistatika und Farbmittel. Für dekorative Zwecke werden die Kunststoffe mit Pigmenten, insbesondere Silberweißpigmenten oder Effektpigmenten, versetzt. Dabei wird häufig insbesondere zwischen den Pigmenten einerseits und Stabilisatoren und Alterungsschutzmitteln andererseits eine unerwünschte Wechselwirkung beobachtet, die vermutlich darin besteht, dass die Stabilisator- und/oder Alterungsmittelmoleküle zu der Oberfläche der Pigmentteilchen diffundieren und dort zu einer Vergilbungsreaktion führen, die vielfach auch im Dunkeln abläuft, insbesondere wenn die Kunststoffe phenolische Komponenten als Antioxidantien, Thermostabilisatoren oder UV-Stabilisatoren enthalten.

Kunststoffe mit phenolischen Bestandteilen zeigen bereits ab einer Pigmentkonzentration von 0,01 Gew.% schon eine Vergilbung. Insbesondere wenn sterisch leicht zugängliche Phenolverbindungen im Kunststoff enthalten sind, kann sich die Vergilbungsreaktion schon bei der Verarbeitung zeigen. Bei sterisch schwer zugänglichen Verbindungen dagegen, tritt die Vergilbung manchmal erst 18 Monate nach der Verarbeitung auf. In der Regel ist die Vergilbungsreaktion bei einer Verarbeitung von 80 °C und einer Pigmentkonzentration von z.B. 0,1 Gew.% innerhalb von 2 h mit dem Auge sichtbar. Die Vergilbungsreaktion führt insbesondere bei Pigmenten mit hellem Farbton zu unschönen Effekten und beeinträchtigt erheblich den ästhetischen Eindruck des Kunststoffsystems. Ursache für die Vergilbungsreaktion ist häufig die Photoaktivität der Titandioxidschicht von Effektpigmenten, welche die photolytische Zersetzung der organischen Bestandteile im Kunststoff oder Lack außerordentlich beschleunigt.

Ein weiteres Problem bei Kunststoffen stellt die Pigmentierung mit Effektpigmenten auf Basis plättchenförmiger Substrate dar, da bei der Einarbeitung der Pigmente in den Kunststoff diese sehr stark beansprucht werden. Die Beanspruchung kann so groß werden, dass die Pigmente brechen und sich dadurch der optische Effekt verändert. Je größer die Plättchen, desto größer ist die Wahrscheinlichkeit, dass die Plättchenform bei der Einarbeitung in den Kunststoff zerstört wird. Insbesondere Effektpigmente mit einem Aspect Ratio (Durchmesser / Plättchendicke) von > 50, ganz besonders bevorzugt von > 100, machen häufig Probleme bei der Einarbeitung.

Aufgabe der vorliegenden Erfindung ist es Perlglanzpigmente, insbesondere Interferenzpigmente und Silberweißpigmente, mit hohem Glanz und mit brillanten Interferenzfarben zur Verfügung zu stellen, die eine erhöhte mechanische Stabilität aufweisen und gleichzeitig lagerstabil sind in Polymeren, insbesondere in phenolhaltigen Kunststoffen und Lacken.

Überraschenderweise wurde nun gefunden, dass Perlglanzpigmente auf der Basis von Glasplättchen, die einen bestimmten Schichtaufbau zeigen und als äußere Schicht eine kalzinierte SiO₂- oder Al₂O₃-Schicht aufweisen, eine deutlich höhere mechanische und chemische Stabilität bei der Einarbeitung in Polymeren, insbesondere Kunststoffen, aufweisen, als beispielsweise Silberweißpigmente auf Basis von Glimmerplättchen.

Gegenstand der vorliegenden Erfindung sind daher Perlglanzpigmente auf der Basis von Glasplättchen, die sich dadurch auszeichnen, dass sie folgenden Schichtaufbau aufweisen:
(A) optional eine Schicht aus SiO₂,
(B) eine hochbrechende Beschichtung mit einem Brechungsindex n > 1,8, die im wesentlichen aus TiO₂ besteht,
(C) eine niedrigbrechende Schicht enthaltend SiO₂ oder Al₂O₃
   und optional
(D) eine äußere Schutzschicht.

Perlglanzpigmente auf der Basis von Glasplättchen sind beispielsweise aus der WO 2004/055119 A und WO 03/006558 A bekannt. Mit TiO₂ beschichtete Glimmerplättchen mit einer finalen Beschichtung aus einem Gemisch aus SiO₂ und Al₂O₃ sind aus der EP 0 644 242 A2 bekannt. Im Stand der Technik werden aber keine Perlglanzpigmente offenbart, die als äußere Beschichtung eine SiO₂- oder Al₂O₃-Schicht aufweisen.

Die erfindungsgemäßen Pigmente zeichnen sich nicht nur durch ihre optischen Effekte aus, sondern zeigen in Polymeren, insbesondere in phenolhaltigen Kunststoffen und Lacken, eine deutlich verbesserte Lagerstabilität. Weiterhin zeichnen sich die Pigmente durch eine erhöhte mechanische Stabilität aus. Im Vergleich zu Silberweißpigmenten auf Basis von Glimmerplättchen wird im Kunststoff keine oder nur eine geringe Hell-/Dunkel-Vergilbung beobachtet, d.h. die erfindungsgemäßen Pigmente zeigen keine und nur eine geringe Oberflächenreaktion mit dem Kunststoff.

Gegenstand der Erfindung ist weiterhin die Verwendung der erfindungsgemäßen Perlglanzpigmente in Farben, Lacken, insbesondere Automobillacken, Pulverlacken, Druckfarben, Sicherheitsdruckfarben, Kunststoffen, keramischen Materialien, Gläsern, Papier, in Tonern für elektrophotographische Druckverfahren, im Saatgut, in Gewächshausfolien und Zeltplanen, als Absorber bei der Lasermarkierung von Papier und Kunststoffen, als Absorber beim Laserschweißen von Kunststoffen und in kosmetischen Formulierungen. Weiterhin sind die erfindungsgemäßen Pigmente auch zur Herstellung von Pigmentanteigungen mit Wasser, organischen und/oder wässrigen Lösemitteln, Pigmentpräparationen sowie zur Herstellung von Trockenpräparaten, wie z. B. Granulaten, Chips, Pellets, Briketts, etc., geeignet. Die Trockenpräparate sind insbesondere für Druckfarben und in der Kosmetik geeignet.

Geeignete Basissubstrate sind aufgrund ihrer glatten Oberflächen und ihres sehr hohen Reflexionsvermögen Glasplättchen. Die Größe der Basissubstrate ist an sich nicht kritisch und kann auf den jeweiligen Anwendungszweck abgestimmt werden. Besonders bevorzugt sind Glasplättchen mit einer durchschnittlichen Dicke von < 2 µm. Dickere Plättchen können in den gängigen Druckverfahren und bei anspruchsvollen Lackierungen in der Regel nicht eingesetzt werden. Vorzugsweise besitzen die Glasplättchen Dicken von < 1 µm, insbesondere von < 0,9 µm, ganz besonders bevorzugt von < 0,7 µm. Insbesondere bevorzugt sind Glasplättchen mit Dicken von 0,25-0,7 µm. Der Durchmesser der Glasplättchen liegt vorzugsweise bei 5-300 µm, insbesondere bevorzugt bei 10-100 µm, ferner bei 5-60 µm. Glasplättchen mit diesen Dimensionen können z. B. nach dem in der EP 0 289 240 beschriebenen Verfahren hergestellt werden.

Die Glasplättchen können aus allen dem Fachmann bekannten Glastypen bestehen, wie z.B. Fensterglas, C-Glas, E-Glas, ECR-Glas, Duran^{®}-Glas, Laborgeräteglas oder optisches Glas. Insbesondere bevorzugt ist E-Glas oder ECR-Glas. Der Brechungsindex der Glasplättchen liegt vorzugsweise bei 1,45-1,80, insbesondere bei 1,50-1,70.

Die chemische Zusammensetzung der Glasplättchen ist bei einer Belegung mit einer SiO₂-Schicht (Schicht (A)) allerdings von untergeordneter Bedeutung für die weiteren Beschichtungen und die resultierenden anwendungstechnischen Eigenschaften der Pigmente. Durch die SiO₂-Belegung wird die Glasoberfläche vor chemischer Veränderung wie Quellung, Auslaugen von Glasbestandteilen oder Auflösung in den aggressiven sauren Belegungslösungen geschützt.

Unter hochbrechenden Beschichtungen sind Schichten mit einem Brechungsindex von > 1,8, unter niedrigbrechenden Schichten solche mit n ≤ 1,8 zu verstehen.

Die Dicke der Schicht (A) auf dem Substrat kann in Abhängigkeit vom gewünschten Effekt in weiten Bereichen variiert werden. Die Schicht (A) weist Dicken von 2-350 nm, vorzugsweise von 5-200 nm, auf. Für die Steuerung von Glanz und Farbstärke sind Schichtdicken von 20-150 nm bevorzugt:

Bei der Schicht (B) handelt es sich vorzugsweise um eine TiO₂-Schicht, die sowohl in der Rutil- als auch in der Anatasmodifikation vorliegen kann, vorzugsweise handelt es sich um eine Rutilschicht. Die TiO₂-Schicht kann aber auch mit Ruß und/oder organischen oder anorganischen Farbmitteln dotiert sein, wobei der Anteil der Dotierung 10 Gew.% bezogen auf die TiO₂-Schicht nicht überschreiten sollte. Die Herstellung von Rutil erfolgt vorzugsweise nach dem Verfahren aus der EP 0 271 767. Die Dicke der hochbrechenden TiO₂-Schicht (B) richtet sich nach der gewünschten Interferenzfarbe und der Dicke der Glasplättchen. Vorzugsweise beträgt die Dicke der Schicht (B) 20-300 nm, vorzugsweise 30-150 nm und insbesondere 40-100 nm. Durch die Kombination der dünnen SiO₂-Schicht, sofern vorhanden, mit einer hochbrechenden Metalloxidschicht lassen sich beispielsweise Interferenzfarben von reinem Silberweiß über Gold bis zu einem intensiven Grün erhalten.

Sofern es sich bei der Schicht (B) um eine Schicht handelt, die im wesentlichen aus Rutil besteht, erfolgt vorzugsweise vor der Belegung mit TiO₂ eine flächendeckende oder partielle Belegung mit SnO₂ oder eine partielle Belegung mit SnO₂-Keimen. Diese sehr dünne SnO₂-Schicht weist Dicken von maximal 10 nm, vorzugsweise ≤ 5 nm, auf.

Die niedrigbrechende Beschichtung (C) besteht vorzugsweise aus ein oder mehreren niedrigbrechenden Oxiden, insbesondere aus SiO₂ oder Al₂O₃, vorzugsweise aus SiO₂. Die Schicht (C) kann 0,005 - 10 Gew.%, vorzugsweise 0,01 - 8 Gew.%, insbesondere 0,05 - 5 Gew.% an weiteren Oxiden aus der Gruppe V, Zr, Zn, Ce, Ti, B, Na, K, Mg, Ca und/oder Mn enthalten. Von den genannten Oxiden sind insbesondere bevorzugt die Oxide von V, Zr, Ce und/oder Zn. Die Dicke der finalen Schicht (C) beträgt 2-200 nm, vorzugsweise 10-80 nm, insbesondere 10-60 nm.

Besonders bevorzugte Perlglanzpigmente werden nachfolgend genannt:
Glasplättchen + SiO₂ + TiO₂ + SiO₂
Glasplättchen + SiO₂ + TiO₂ + Al₂O₃
Glasplättchen + TiO₂ + SiO₂
Glasplättchen + TiO₂ + Al₂O₃

Die erfindungsgemäßen Perlglanzpigmente lassen sich in der Regel relativ leicht herstellen.

Die Metalloxidschichten werden vorzugsweise nasschemisch aufgebracht, wobei die zur Herstellung von Perlglanzpigmenten entwickelten nasschemischen Beschichtungsverfahren angewendet werden können. Derartige Verfahren sind z.B. beschrieben in DE 14 67 468, DE 19 59 988, DE 20 09 566, DE 22 14 545, DE 22 15 191, DE 22 44, 298, DE 23 13 331, DE 15 22 572, DE 31 37 808, DE 31 37 809, DE 31 51 343, DE 31 51 354, DE 31 51 355, DE 32 11 602, DE 32 35 017 oder auch in weiteren dem Fachmann bekannten Patentdokumenten und sonstigen Publikationen.

Bei der Nassbeschichtung werden die Substratpartikel in Wasser suspendiert und mit ein oder mehreren hydrolysierbaren Metallsalzen oder einer Wasserglaslösung bei einem für die Hydrolyse geeigneten pH-Wert versetzt, der so gewählt wird, dass die Metalloxide bzw. Metalloxidhydrate direkt auf den Plättchen ausgefällt werden, ohne dass es zu Nebenfällungen kommt. Der pH-Wert wird üblicherweise durch gleichzeitiges Zudosieren einer Base und/oder Säure konstant gehalten. Anschließend werden die Pigmente abgetrennt, gewaschen und bei 50-150 °C für 6-18 h getrocknet und 0,5-3 h geglüht, wobei die Glühtemperatur im Hinblick auf die jeweils vorliegende Beschichtung optimiert werden kann. In der Regel liegen die Glühtemperaturen zwischen 500 und 1000 °C, vorzugsweise zwischen 600 und 900 °C. Falls gewünscht, können die Pigmente nach Aufbringen einzelner Beschichtungen abgetrennt, getrocknet und ggf. geglüht werden, um dann zur Auffällung der weiteren Schichten wieder resuspendiert zu werden.

Die Auffällung der SiO₂-Schicht auf das Substrat erfolgt in der Regel durch Zugabe einer Kalium- oder Natronwasserglas-Lösung bei einem geeigneten pH-Wert.

Weiterhin kann die Beschichtung auch in einem Wirbelbettreaktor durch Gasphasenbeschichtung erfolgen, wobei z. B. die in EP 0 045 851 und EP 0 106 235 zur Herstellung von Perlglanzpigmenten vorgeschlagenen Verfahren entsprechend angewendet werden können.

Der Farbton der Perlglanzpigmente kann in sehr weiten Grenzen durch die unterschiedliche Wahl der Belegungsmengen bzw. der daraus resultierenden Schichtdicken variiert werden. Die Feinabstimmung für einen bestimmten Farbton kann über die reine Mengenwahl hinaus durch visuell oder messtechnisch kontrolliertes Anfahren der gewünschten Farbe erreicht werden.

Zur Erhöhung der Licht-, Wasser- und Wetterstabilität empfiehlt es sich häufig, in Abhängigkeit vom Einsatzgebiet, das fertige Pigment einer Nachbeschichtung oder Nachbehandlung zu unterziehen. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage. Durch diese Nachbeschichtung (Schicht D) wird die chemische und photochemische Stabilität weiter erhöht oder die Handhabung des Pigments, insbesondere die Einarbeitung in unterschiedliche Medien, erleichtert. Zur Verbesserung der Benetzbarkeit, Dispergierbarkeit und/oder Verträglichkeit mit den Anwendermedien können beispielsweise funktionelle Beschichtungen aus Al₂O₃ oder ZrO₂ oder deren Gemische auf die Pigmentoberfläche aufgebracht werden. Weiterhin sind organische Nachbeschichtungen möglich, z.B. mit Silanen, wie beispielsweise beschrieben in der EP 0 090259, EP 0 634 459, WO 99/57204, WO 96/32446, WO 99/57204, U.S. 5,759,255, U.S. 5,571,851, WO 01/92425 oder in J.J. Ponjeé, Philips Technical Review, Vol. 44, No. 3, 81 ff. und P.H. Harding J.C. Berg, J. Adhesion Sci. Technol. Vol. 11 No. 4, S. 471-493.

Die erfindungsgemäßen Pigmente sind mit einer Vielzahl von Farbsystemen kompatibel, vorzugsweise aus dem Bereich der Lacke, Farben, Druckfarben und kosmetischen Formulierungen. Für die Herstellung der Druckfarben, z. B. für den Tiefdruck, Flexodruck, Offsetdruck, Offsetüberdrucklackierung, ist eine Vielzahl von Bindern, insbesondere wasserlösliche Typen, geeignet, wie sie z. B. von den Firmen BASF, Marabu, Pröll, Sericol, Hartmann, Gebr. Schmidt, Sicpa, Aarberg, Siegberg, GSB-Wahl, Follmann, Ruco oder Coates Screen INKS GmbH vertrieben werden. Die Druckfarben können auf Wasserbasis oder Lösemittelbasis aufgebaut sein. Weiterhin sind die Pigmente auch für die Lasermarkierung von Papier und Kunststoffen sowie für die Anwendungen im Agrarbereich, z. B. für Gewächshausfolien, sowie z. B. für die Farbgebung von Zeltplanen, geeignet.

Besonders geeignet sind die erfindungsgemäßen Perlglanzpigmente infolge ihrer erhöhten chemischen und mechanischen Stabilisierung für Polymere, insbesondere Kunststoffe. Die Kunststoffe enthalten vorzugsweise 0,01-10 Gew.%, insbesondere 0,3-5 Gew.% und ganz besonders bevorzugt 0,5-2,5 Gew.%, der erfindungsgemäßen Pigmente. Selbst bei Pigmentkonzentrationen von 5 Gew.% zeigt sich keine signifikante Dunkelvergilbung von Kunststoffen enthaltend phenolhaltige Additive. Weiterhin sind die Perlglanzpigmente für Lackformulierungen geeignet.

Da die erfindungsgemäßen Perlglanzpigmente eine besonders klare Farbe mit intensiven Interferenzfarben und hoher Brillanz verbinden, lassen sich mit ihnen besonders wirksame Effekte in den verschiedenen Anwendungsmedien erzielen, z. B. in kosmetischen Formulierungen, wie Nagellacken, Lippenstiften, Presspudern, Gelen, Lotionen, Emulsionen, Seifen, Zahnpasten.

Die erfindungsgemäßen Pigmente können weiterhin auch mit kosmetischen Wirkstoffen kombiniert werden. Geeignete Wirkstoffe sind z.B. Insect Repellents, UV A/BC-Schutzfilter (z.B. OMC, B3, MBC), Anti-Ageing-Wirkstoffe, Vitamine und deren Derivate (z.B. Vitamin A, C, E, etc.), Selbstbräuner (z.B. DHA, Erytrolyse, u.a.) sowie weitere kosmetische Wirkstoffe, wie z.B. Bisabolol, LPO, Ectoin, Emblica, Allantoin, Bioflavanoide und deren Derivate.

Bei Selbstbräunungscremes, -lotionen, -sprays, etc. enthaltend beispielsweise den Selbstbräuner DHA (Dihydroxyaceton) und ein Effektpigment mit abschließender TiO₂-Schicht, z.B. ein mit TiO₂ (Anatas) beschichtetes Glasplättchen, wird das DHA langsam in der Formulierung abgebaut. Bei Verwendung der erfindungsgemäßen Pigmente in der Formulierung bleibt das DHA in seiner Wirkung voll erhalten.

Die die erfindungsgemäßen Perlglanzpigmente enthaltenden Formulierungen können dem lipophilen, hydrophilen oder hydrophoben Typ angehören. Bei heterogenen Formulierungen mit diskreten wässrigen und nichtwässrigen Phasen können die erfindungsgemäßen Pigmente in jeweils nur einer der beiden Phasen enthalten oder auch über beide Phasen verteilt sein.

Es versteht sich von selbst, dass für die verschiedenen Anwendungszwecke die erfindungsgemäßen Perlglanzpigmente auch vorteilhaft in Abmischung mit organischen Farbstoffen, organischen Pigmenten oder anderen Pigmenten, wie z.B. transparenten und deckenden Weiß-, Bunt- und Schwarzpigmenten sowie mit plättchenförmigen Eisenoxiden, organischen Pigmenten, holographischen Pigmenten, LCPs (Liquid Crystal Polymers) und herkömmlichen transparenten, bunten und schwarzen Glanzpigmenten auf der Basis von metalloxidbeschichteten Glimmer- und SiO₂-Plättchen, etc., verwendet werden können. Die erfindungsgemäßen Pigmente können in jedem Verhältnis mit handelsüblichen Pigmenten und Füllern gemischt werden.

Als Füllstoffe sind z. B. zu nennen natürlicher und synthetischer Glimmer, Nylon Powder, reine oder gefüllte Melaminharze, Talcum, SiO₂, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, sowie physikalische oder chemische Kombinationen dieser Stoffe.

Bezüglich der Partikelform des Füllstoffes gibt es keine Einschränkungen. Sie kann den Anforderungen gemäß z. B. plättchenförmig, sphärisch oder nadelförmig sein.

Selbstverständlich können die erfindungsgemäßen Pigmente in den Formulierungen auch mit jeder Art von Roh- und Hilfsstoffen kombiniert werden. Dazu gehören u.a. Öle, Fette, Wachse, Filmbildner, Konservierungsmittel und allgemein anwendungstechnische Eigenschaften bestimmende Hilfsstoffe, wie z. B. Verdickter und rheologische Zusatzstoffe wie etwa Bentonite, Hektorite, Siliciumdioxide, Ca-Silicate, Gelatinen, hochmolekulare Kohlenhydrate und/oder oberflächenaktive Hilfsmittel, etc.

Gegenstand der Erfindung ist somit auch die Verwendung der Perlglanzpigmente in Formulierungen wie Farben, Lacken, Automobillacken, Pulverlacken, Druckfarben, Sicherheitsdruckfarben, Kunststoffen, keramischen Materialien, Gläsern, Papier, im Papierstrich, in Tonern für elektrophotographische Druckverfahren, im Saatgut, in Gewächshausfolien und Zeltplanen, als Absorber bei der Lasermarkierung von Papier und Kunststoffen, als Absorber beim Laserschweißen von Kunststoffen, kosmetischen Formulierungen, zur Herstellung von Pigmentanteigungen mit Wasser, organischen und/oder wässrigen Lösemitteln, zur Herstellung von Pigmentpräparationen und Trocken-präparaten, wie z. B. Granulaten. Die nachfolgenden Beispiele sollen die Erfindung näher erläutern ohne sie jedoch zu beschränken.

### Beispiele

### I. Herstellung der Pigmente

### Beispiel 1: Glasplättchen + SiO₂ + TiO₂ (Rutil) + SiO₂

150 g Glasplättchen mit einer mittleren Schichtdicke von 900 nm der Fraktion 20-200 µm werden in 1,9 I VE-Wasser unter Rühren auf 75 °C erhitzt. Nun wird mit einer 5 %igen Salzsäure der pH-Wert der Suspension auf 7,5 eingestellt. Anschließend wird eine Natronwasserglaslösung (112 g Natronwasserglaslösung enthaltend 26,8 % SiO₂ gelöst in 112 g VE-Wasser) zugetropft, wobei der pH-Wert durch gleichzeitiges Zudosieren einer 5 %igen Salzsäure konstant bei 7,5 gehalten wird. Nach vollständiger Zugabe wird 0,5 h nachgerührt. Dann wird der pH-Wert der Suspension auf 1,8 eingestellt, 15 Minuten nachgerührt und eine salzsaure Zinntetrachlorid-Lösung (3 g SnCl₄ * 5 H₂O, gelöst in 15 ml 25 %iger Salzsäure und 85 ml voll entsalztem Wasser) zugetropft, wobei der pH-Wert durch gleichzeitiges Zutropfen einer 32 %igen Natronlauge konstant gehalten wird. Nach vollständiger Zugabe wird 15 Minuten nachgerührt.

Es folgt ein Zudosieren einer 30 %igen Titantetrachloridlösung, wobei der pH-Wert durch gleichzeitiges Zutropfen einer 32 %igen Natronlauge konstant gehalten wird. Durch Farbmessung während des Prozesses wird die Koloristik während der Herstellung des Pigments kontrolliert und der Fällungsprozess nach dem Buntton (Bunttonwinkel arc tan b*/a*) gesteuert. Nach Erreichen des gewünschten Silber-Endpunktes wird 15 Minuten nachgerührt.

Der pH-Wert wird mit verdünnter Natronlauge auf pH 9,0 eingestellt. Innerhalb von 2 Stunden werden 100 ml einer Natriumwasserglaslösung mit einem Kieselsäuregehalt von 5 % zugegeben, wobei der pH-Wert mittels 2,5 %iger Schwefelsäure konstant gehalten wird. Anschließend wird 30 Minuten nachgerührt und dann der pH-Wert mit Schwefelsäure innerhalb von 30 Minuten auf 7,5 gestellt.

Nach einer Nachrührzeit von 30 Minuten wird das nachbeschichtete Pigment durch Filtration vom Überstand abgetrennt und gewaschen. Nach Trocknung bei 100 bis 150 °C wird das Pigment 45 Minuten bei 700 °C calciniert und entsprechend der gewünschten Korngröße gesiebt.

Das so erhaltene Pigment enthält eine Beschichtung von Siliziumdioxid mit einer Schichtdicke von ca. 18-28 nm.

### Beispiel 2A: Glasplättchen + TiO₂ (Anatas) + SiO₂

150 g Glasplättchen mit einer mittleren Schichtdicke von 900 nm der Fraktion 20-200 µm werden in 1,9 I VE-Wasser unter Rühren auf 75 °C erhitzt. Nun wird mit konz. Salzsäure der pH-Wert der Suspension auf 2,0 eingestellt.

Es folgt ein Zudosieren einer 30 %igen Titantetrachloridlösung, wobei der pH-Wert durch gleichzeitiges Zutropfen einer 32 %igen Natronlauge konstant gehalten wird. Durch Farbmessung während des Prozesses wird die Koloristik während der Herstellung des Pigments kontrolliert und der Fällungsprozess nach dem Buntton (Bunttonwinkel arc tan b*/a*) gesteuert. Nach Erreichen des gewünschten Silber-Endpunktes wird 15 Minuten nachgerührt.

Der pH-Wert wird mit verdünnter Natronlauge auf pH 9,0 eingestellt. Innerhalb von 2 Stunden werden 100 ml einer Natriumwasserglaslösung mit einem Kieselsäuregehalt von 5 % zugegeben, wobei der pH-Wert mittels 2,5 %iger Schwefelsäure konstant gehalten wird. Anschließend wird 30 Minuten nachgerührt und dann der pH-Wert mit Schwefelsäure innerhalb von 30 Minuten auf 7,5 gestellt.

Nach einer Nachrührzeit von 30 Minuten wird das nachbeschichtete Pigment durch Filtration vom Überstand abgetrennt und gewaschen. Nach Trocknung bei 100 bis 150 °C wird das Pigment 45 Minuten bei 700 °C calciniert.

Das so erhaltene Pigment enthält eine Beschichtung von Siliziumdioxid mit einer Schichtdicke von ca. 18-28 nm.

### Beispiel 2 B: Glasplättchen + TiO₂ (Rutil) + SiO₂

150 g Glasplättchen mit einer mittleren Schichtdicke von 900 nm der Fraktion 20-200 µm werden in 1,9 I VE-Wasser unter Rühren auf 75 °C erhitzt. Nun wird der pH-Wert der Suspension auf 2,0 eingestellt, 15 Minuten nachgerührt und eine salzsaure Zinntetrachlorid-Lösung (3 g SnCl₄ * 5 H₂O, gelöst in 15 ml 25 %iger Salzsäure und 85 ml voll entsalztem Wasser) zugetropft, wobei der pH-Wert durch gleichzeitiges Zutropfen einer 32 %igen Natronlauge konstant gehalten wird. Nach vollständiger Zugabe wird 15 Minuten nachgerührt.

Es folgt ein Zudosieren einer 30 %igen Titantetrachloridlösung, wobei der pH-Wert durch gleichzeitiges Zutropfen einer 32 %igen Natronlauge konstant gehalten wird. Durch Farbmessung während des Prozesses wird die Koloristik während der Herstellung des Pigments kontrolliert und der Fällungsprozess nach dem Buntton (Bunttonwinkel arc tan b*/a*) gesteuert. Nach Erreichen des gewünschten Silber-Endproduktes wird 15 Minuten nachgerührt.

Der pH-Wert wird mit verdünnter Natronlauge auf pH 9,0 eingestellt. Innerhalb von 2 Stunden werden 100 ml einer Natriumwasserglaslösung mit einem Kieselsäuregehalt von 5 % zugegeben, wobei der pH-Wert mittels 2,5 %iger Schwefelsäure konstant gehalten wird. Anschließend wird 30 Minuten nachgerührt und dann der pH-Wert mit Schwefelsäure innerhalb von 30 Minuten auf 7,5 gestellt.

Nach einer Nachrührzeit von 30 Minuten wird das nachbeschichtete Pigment durch Filtration vom Überstand abgetrennt und gewaschen. Nach Trocknung bei 100 bis 150 °C wird das Pigment 45 Minuten bei 700 °C calciniert.

Das so erhaltene Pigment erhält eine Beschichtung von Siliziumdioxid mit einer Schichtdicke von ca. 18-28 nm.

### Beispiel 3: Glasplättchen + TiO₂ + Al₂O₃

150 g Glasplättchen mit einer mittleren Schichtdicke von 900 nm der Fraktion 20-200 µm werden in 1,9 I VE-Wasser unter Rühren auf 75 °C erhitzt. Nun wird mit konz. Salzsäure der pH-Wert der Suspension auf 2,0 eingestellt.

Es folgt ein Zudosieren einer 30 %igen Titantetrachloridlösung, wobei der pH-Wert durch gleichzeitiges Zutropfen einer 32 %igen Natronlauge konstant gehalten wird. Durch Farbmessung während des Prozesses wird die Koloristik während der Herstellung des Pigments kontrolliert und der Fällungsprozess nach dem Buntton (Bunttonwinkel arc tan b*/a*) gesteuert. Nach Erreichen des gewünschten Silber-Endpunktes wird 15 Minuten nachgerührt.

Die erhaltene Pigmentsuspension wird mit 32 % NaOH auf pH 6,5 eingestellt. Nach 15 Minuten Rühren werden 5,33 g AlCl₃ x 6 H₂O und 3,20 g Natriumsulfat in fester Form zugegeben, Die erhaltene Suspension wird 1 h bei 90 °C gerührt. Anschließend wird die wässrige Lösung abgesaugt, chloridfrei gewaschen und das Produkt getrocknet Das Produkt wird 30 Minuten bei 700 °C geglüht.

### Beispiel 4: Glasplättchen + TiO₂ + SiO₂ (dotiert mit K⁺)

150 g Glasplättchen mit einer mittleren Schichtdicke von 900 nm der Fraktion 20 - 200 µm werden in 1,9 I VE-Wasser unter Rühren auf 75 °C erhitzt. Nun wird mit konz. Salzsäure der pH-Wert der Suspension auf 2,0 eingestellt.

Es folgt ein Zudosieren einer 30 %igen Tiantetrachloridlösung, wobei der pH-Wert durch gleichzeitiges Zutropfen einer konz. Kalilauge konstant gehalten wird. Durch Farbmessung während des Prozesses wird die Koloristik während der Herstellung des Pigments kontrolliert und der Fällungsprozess nach dem Buntton (Bunttonwinkel arc tan b*/a*) gesteuert. Nach Erreichen des gewünschten Silber-Endpunktes wird 15 Minuten nachgerührt.

Der pH-Wert wird mit verdünnter Kalilauge auf pH 9,0 eingestellt. Innerhalb von 2 Stunden werden 100 ml einer Kaliumwasserglaslösung mit einem Kieselsäuregehalt von 5 % zugegeben, wobei der pH-Wert mittels 2,5 %iger Schwefelsäure konstant gehalten wird. Anschließend wird 30 Minuten nachgerührt und dann der pH-Wert mit Schwefelsäure innerhalb von 30 Minuten auf 7,5 gestellt.

Nach einer Nachrührzeit von 30 Minuten wird das nachbeschichtete Pigment durch Filtration vom Überstand abgetrennt und gewaschen. Nach Trocknung bei 100 bis 150 °C wird das Pigment 45 Minuten bei 700 °C calciniert.

Das so erhaltene Pigment enthält eine Beschichtung von Siliziumdioxid mit einer Schichtdicke von ca. 18-28 nm.

### Beispiel 5 (Vergleichsbeispiel für ein Pigment, das Vergilbung zeigt): Glasplättchen + TiO₂ (Rutil)

150 g Glasplättchen mit einer mittleren Schichtdicke von 900 nm der Fraktion 20 - 200 µm werden in 1,9 I VE-Wasser unter Rühren auf 75 °C erhitzt. Nun wird der pH-Wert der Suspension auf 2,0 eingestellt, 15 Minuten nachgerührt und eine salzsaure Zinntetrachloridlösung (3 g SnCl₄ * 5 H₂O, gelöst in 15 ml 25 %iger Salzsäure und 85 ml voll entsalztem Wasser) zugetropft, wobei der pH-Wert durch gleichzeitiges Zutropfen einer 32 %igen Natronlauge konstant gehalten wird. Nach vollständiger Zugabe wird 15 Minuten nachgerührt.

Es folgt ein Zudosieren einer 30 %igen Titantetrachloridlösung, wobei der pH-Wert durch gleichzeitiges Zutropfen einer 32 %igen Natronlauge konstant gehalten wird. Durch Farbmessung während des Prozesses wird die Koloristik während der Herstellung des Pigments kontrolliert und der Fällungsprozess nach dem Buntton (Bunttonwinkel arc tan b*/a*) gesteuert. Nach Erreichen des gewünschten Silber-Endpunktes wird 15 Minuten nachgerührt.

Nach einer Nachrührzeit von 30 Minuten wird das nachbeschichtete Pigment durch Filtration vom Überstand abgetrennt und gewaschen. Nach Trocknung bei 100 bis 150 °C wird das Pigment 45 Minuten bei 700 °C calciniert.

### II. Stabilitätsuntersuchunaen

### 2.1 Vergilbung (Kunststoff)

Das Phänomen "Vergilbung" ist chemisch nicht einfach zu beschreiben. Der Effekt wird unter anderem beim Einsatz von TiO₂-haltigen Pigmenten beobachtet, die zusammen mit phenolischen Substanzen, je nach deren Struktur unter verschiedenen Umgebungseinflüssen, gelbliche Komplexe bilden. Das Ergebnis der "Vergilbung" ist die Änderung der Farbe der Probe in Richtung gelb. Da sich die Farbe im Gelbton ändert, kann bei farbmetrischer Beschreibung der "Vergilbung" im Lab-System am besten der b-Wert herangezogen werden.

In thermoplastischen Kunststoffen wird diese Vergilbung am Beispiel 6 der folgenden Einfärbungen beobachtet, während Beispiel 7 den erfindungsgemäßen Zustand ohne Vergilbung zeigt. Besonders auffällig ist die Vergilbung natürlich bei sehr hellen oder silberweißen Farben.

### Beispiel 6:

1,6 kg PE-LLD Pulver (Escorene 6101 RQ von Fa. Exxon) werden mit 400 g Pigment gemäß Beispiel 6 in einem geeigneten Mischer 10 Minuten gemischt und anschließend auf einem Doppelschneckenextruder aufgeschmolzen, gemischt und über eine Granuliereinrichtung zu einem silbrig glänzenden Masterbatchgranulat verarbeitet.

Dieses Masterbatch verändert seine silbrig weiße Farbe beim Lagern in einem mäßig hell beleuchteten Lagerraum in Laufe von zwei Wochen an der dem Licht zugewandten Seite deutlich nach gelb. Nach dem Graumaßstab (DIN EN 20105-A02) beurteilt, ergibt sich eine Bewertung von 2 G (gelb).

### Bespiel 7:

1,6 kg PE-LLD Pulver (Escorene 6101 RQ von Fa. Exxon) werden mit 400 g eines erfindungsgemäßen Pigments (nach Beispiel 2B hergestellt) in einem geeigneten Mischer 10 Minuten gemischt und anschließend auf einem Doppelschneckenextruder aufgeschmolzen, gemischt und über eine Granuliereinrichtung zu einem silbrig glänzenden Masterbatchgranulat verarbeitet.

Dieses Masterbatch ändert beim Lagern in einem mäßig hell beleuchteten Lagerraum in Laufe von zwei Wochen seine silbrig weiße Farbe nicht.

Nach dem Graumaßstab (DIN EN 20105-A02) beurteilt, ergibt sich eine Bewertung von 5, d. h. keine farbliche Veränderung.

### 2.2 Quantitative Bestimmung der Vergilbung

Mit Hilfe der nachfolgenden Methode kann die Vergilbung quantitativ bestimmt werden:
In eine Meßküvette wird 0,1 g der zu testenden Probe gegeben. Hinzu kommen 0,4 ml einer Lösung bestehend aus 5 % Propanol/95 % Dibutylphthalate. Die Suspension wird eine Minute geschüttelt und anschließend der b-Wert mit einem Calorimeter Minolta CR 300 gemessen. Der b-Wert wird unter,dem Winkel 45°/0 bestimmt. Anschließend wird 0,5 ml einer Propylgalat- (Propyl 3,4,5,-Trihydroxybenzoat)-Lösung (10 % in 40 % Propanol/60 % Dibutylphthalate) hinzugefügt. Die Suspension wird 3 Minuten geschüttelt und anschließend 1 Minute stehen gelassen. Der b-Wert wird erneut vermessen und die Differenz des b-Wertes zwischen der ersten und der zweiten Messung bestimmt. Anhand der Änderung des b-Wertes ergibt sich die Vergilbung.

### Beispiel 8:

a) Muster nach Beispiel 6 zeigt Vergilbung: delta b-Wert von + 4,5
b) Muster nach Beispiel 2B zeigt keine Vergilbung: delta b-Wert von + 0,1

### 2.3 Stabilitätsuntersuchung

10 g Perlglanzpigment werden jeweils in 100 ml Wasser suspendiert und durch schnelles Rühren mechanisch belastet.

Die Ergebnisse der mechanischen Stabilitätsuntersuchung bezüglich Größe und Fraktionsverteilung der Pigmente sind nachfolgend in Tabelle 1 zusammengefaßt:

**Tabelle 1:**

| | Unbelastet | Belastet | Unbelastet | Belastet |
|---|---|---|---|---|
| | D₅₀ | D₅₀ | D₉₅ | D₉₅ |
| Pigment gemäß Beispiel 5 (Vergleich) | 82 | 71 | 188 | 147 |
| Pigment gemäß Beispiel 2B (erfindungsgemäß) | 82 | 82 | 179 | 179 |

### Anwendungsbeispiele:

### Anwendungsbeispiel 1: Pflegende Selbstbräunungscreme (O/W)

| Inhaltsstoffe | | INCI | [%] |
|---|---|---|---|
| **Phase A** | | | |
| Montanov 68 | (1) | Cetearyl Alcohol, Cetearyl Glucoside | 4,00 |
| Span 60 | (2) | Sorbitan Stearate | 1,50 |
| Lanette O | (3) | Cetearyl Alcohol | 1,00 |
| Cosmacol ELI | (4) | C12-13 Alkyl Lactate | 3,00 |
| Cosmacol EMI | (4) | DI-C12-13 Alkyl Malate | 1,50 |
| Arlamol HD | (2) | Isohexadecane | 3,00 |
| Dow Corning 9040 Silicone Elastomer Blend | (5) | Cyclomethicone, Dimethicone Crosspolymer | 1,00 |
| RonaCare^{®} Tocopherol Acetate | (6) | Tocopherol Acetate | 0,50 |
| Propyl-4-hydroxybenzoate | (6) | Propylparaben | 0,05 |

| **Phase B** | | | |
|---|---|---|---|
| RonaCare^{®} Ectoin | (6) | Ectoin | 0,50 |
| Pigment nach Beispiel 1 oder Beispiel 2 | | | 2,00 |
| Glycerol, anhydrous | (6) | Glycerin | 2,00 |
| FD&C Yellow No. 6 W082 | (8) | Cl 15985 | 0,01 |
| Methyl-4-hydroxybenzoate | (6) | Methylparaben | 0,15 |
| Water, demineralized | | Aqua (Water) | 64,09 |

| **Phase C** | | | |
|---|---|---|---|
| Sepigel 305 | (1) | Laureth-7, Polyacrylamide, C13-14 Isoparaffin | 0,50 |

| **Phase D** | | | |
|---|---|---|---|
| Dihydroxyacetone | (6) | Dihydroxyacetone | 5,00 |
| Water, demineralized | | Aqua (Water) | 10,00 |

| **Phase E** | | | |
|---|---|---|---|
| Fragrance Babylon | (9) | Parfum | 0,20 |

### Herstellung:

Phase A und B werden separat auf 75 °C erwärmt. Dann wird zu Phase A langsam Phase B unter Rühren zugemischt. Bei 60 °C wird Phase C zu der Phase A/B mit einem Handrührer zugemischt und homogenisiert. Auf 40 °C abkühlen lassen und die Phase D und Phase E unterrühren.

### Bemerkungen:

pH_{23°C} = 4,0
Viskosität: 18,600 cps (Brookfield model RVT DV-II, Helipath Spindle C,
10 rpm) bei 23 °C

### Bezugsquellen:

(1) Seppic
(2) Uniqema
(3) Cognis GmbH
(4) Condea Chimica D.A.C. S.p.A.
(5) Dow Corning
(6) Merck KGaAlRona^{®}
(7) D.D.Williamson
(8) Les Colorants Wackherr SA
(9) Drom

### Anwendungsbeispiel 2: Selbstbräunungscreme (O/W)

| Inhaltsstoffe | | INCI | [%] |
|---|---|---|---|
| **Phase A** | | | |
| Tego Care 150 | (1) | Glyceryl Stearate, Steareth-25, Ceteth-20, Stearyl Alcohol | 8,00 |
| Paraffin liquid | (2) | Parffinum Liquidum (Mineral Oil) | 12,00 |
| Paraffin schüttfähig | (2) | Paraffin | 2,00 |
| Miglyol 812 N | (3) | Caprylic/Capric Triglyceride | 3,00 |
| Isopropyl myristate | (4) | Isopropyl Myristate | 2,00 |
| Propyl-4-hydroxybenzoate | (2) | Propylparaben | 0,15 |

| **Phase B** | | | |
|---|---|---|---|
| 1,2-Propanediol | (2) | Propylene Glycol | 4,00 |
| Sorbitol F liquid | (2) | Sorbitol | 2,00 |
| Water, demineralized | | Aqua (Water) | 47,40 |
| Methyl-4-hydroxybenzoate | (2) | Methylparaben | 0,15 |
| Pigment nach Beispiel 1 oder Beispiel 2 | | | 2,00 |

| **Phase C** | | | |
|---|---|---|---|
| Dihydroxyacetone | (2) | Dihydroxyacetone | 5,00 |
| Water, demineralized | | Aqua (Water) | 11,80 |

| **Phase D** | | | |
|---|---|---|---|
| Fragrance (q.s.) | | Parfum | 0,50 |

### Herstellung:

Phase A wird auf 80 °C erwärmt und Phase B auf 75 °C. Dann wird Phase A unter Rühren langsam zu Phase B gegeben. Mit einem Handrührer wird die Mischung bei 65 °C für eine Minute homogenisiert. Auf 40 °C abkühlen lassen und die Phase C unterrühren. Weiter auf 35 °C abkühlen und Phase D unter Rühren untermischen.

### Bemerkungen:

pH_{23°C} = 4,6
Viskosität: 42,500 mPas (Brookfield RVT, Sp. C, 10 rpm) bei 23 °C

### Bezugsquellen:

(1) Degussa-Goldschmidt AG
(2) Merck KGaA/Rona^{®}
(3) Sasol Germany GmbH
(4) Cognis GmbH

## Patentansprüche

1. Perlglanzpigmente auf der Basis von Glasplättchen, **dadurch gekennzeichnet, dass** sie folgenden Schichtaufbau aufweisen:
(A) optional eine Schicht aus SiO₂,
(B) eine hochbrechende Beschichtung mit einem Brechungsindex n > 1,8, die im wesentlichen aus TiO₂ besteht,
(C) eine niedrigbrechende Schicht enthaltend SiO₂ oder Al₂O₃
und optional
(D) eine äußere Schutzschicht.

2. Perlglanzpigmente nach Anspruch 1, **dadurch gekennzeichnet, dass** die Dicke der Schicht (A) 2-350 nm beträgt.

3. Perlglanzpigmente nach Anspruch 1 oder 2, **dadurch gekennzeichnet, dass** die Dicke der Schicht (B) 20-300 nm beträgt.

4. Perlglanzpigmente nach einem der Ansprüche 1 bis 3, **dadurch gekennzeichnet, dass** die Dicke der Schicht (C) 2-200 nm beträgt.

5. Perlglanzpigmente nach einem der Ansprüche 1 bis 4, **dadurch gekennzeichnet, dass** die Schicht (C) zusätzlich mit ein oder mehreren Oxiden aus der Gruppe V, Zr, Zn, Ce, Ti, B, Na, K, Mg, Ca und/oder Mn dotiert ist.

6. Perlglanzpigmente nach einem der Ansprüche 1 bis 5, **dadurch gekennzeichnet, dass** das TiO₂ der Schicht (B) in der Rutilmodifikation vorliegt.

7. Perlglanzpigmente nach Anspruch 6, **dadurch gekennzeichnet, dass** vor der Belegung mit TiO₂ eine Zwischenschicht aus SnO₂ bzw. SnO₂-Keimen aufgebracht wird.

8. Perlglanzpigmente nach einem der Ansprüche 1 bis 7, **dadurch gekennzeichnet, dass** sie folgenden Schichtaufbau besitzen;
Glasplättchen + SiO₂ + TiO₂ + SiO₂
Glasplättchen + SiO₂ + TiO₂ + Al₂O₃
Glasplättchen + TiO₂ + SiO₂
Glasplättchen + TiO₂ + Al₂O₃

9. Perlglanzpigmente nach einem der Ansprüche 1 bis 8, **dadurch gekennzeichnet, dass** sie zu Erhöhung der Licht-, Temperatur- und Wetterstabilität eine äußere Schutzschicht (D) aufweisen.

10. Verfahren zur Herstellung der Perlglanzpigmente nach einem der Ansprüche 1 bis 9, **dadurch gekennzeichnet, dass** die Beschichtung der Glasplättchen nasschemisch durch hydrolytische Zersetzung von Metallsalzen in wässrigem Medium oder im Wirbelbettreaktor durch Gasphasenbeschichtung erfolgt

11. Verwendung der Perlglanzpigmente nach einem der Ansprüche 1 bis 9 in Farben, Lacken, Automobillacken, Pulverlacken, Druckfarben, Sicherheitsdruckfarben, Kunststoffen, keramischen Materialien, Gläsern, Papier, in Tonern für elektrophotographische Druckverfahren, im Saatgut, in Gewächshausfolien und Zeltplanen, als Absorber bei der Lasermarkierung von Papier und Kunststoffen, als Absorber beim Laserschweißen von Kunststoffen, in kosmetischen Formulierungen, zur Herstellung von Pigmentanteigungen mit Wasser, organischen und/oder wässrigen Lösemitteln, zur Herstellung von Pigmentpräparationen und Trockenpräparaten.

12. Verwendung der Perlglanzpigmente nach einem der Ansprüche 1 bis 9 zur Vergilbungsinhibierung von Polymeren enthaltend phenolische Additive.

13. Verwendung der Perlglanzpigmente nach Anspruch 11 in kosmetischen Formulierungen enthaltend Dihydroxyaceton.

## Claims

1. Pearlescent pigments based on glass flakes, **characterised in that** they have the following layer structure:
(A) optionally a layer of SiO₂,
(B) a high-refractive-index coating having a refractive index n > 1.8 which essentially consists of TiO₂,
(C) a low-refractive-index layer comprising SiO₂ or Al₂O₃
and optionally
(D) an outer protective layer.

2. Pearlescent pigments according to Claim 1, **characterised in that** the thickness of layer (A) is 2-350 nm.

3. Pearlescent pigments according to Claim 1 or 2, **characterised in that** the thickness of layer (B) is 20-300 nm.

4. Pearlescent pigments according to one of Claims 1 to 3, **characterised in that** the thickness of layer (C) is 2-200 nm.

5. Pearlescent pigments according to one of Claims 1 to 4, **characterised in that** layer (C) is additionally doped with one or more oxides from the group V, Zr, Zn, Ce, Ti, B, Na, K, Mg, Ca and/or Mn.

6. Pearlescent pigments according to one of Claims 1 to 5, **characterised in that** the TiO₂ in layer (B) is in the rutile modification.

7. Pearlescent pigments according to Claim 6, **characterised in that**, before the coating with TiO₂, an interlayer of SnO₂ or SnO₂ nuclei is applied.

8. Pearlescent pigments according to one of Claims 1 to 7, **characterised in that** they have the following layer structure:
glass flake + SiO₂ + TiO₂ + SiO₂
glass flake + SiO₂ + TiO₂ + Al₂O₃
glass flake + TiO₂ + SiO₂
glass flake + TiO₂ + Al₂O₃.

9. Pearlescent pigments according to one of Claims 1 to 8, **characterised in that** they have an outer protective layer (D) in order to increase the light, temperature and weather stability.

10. Process for the preparation of the pearlescent pigments according to one of Claims 1 to 9, **characterised in that** the coating of the glass flakes is carried out by wet-chemical methods by hydrolytic decomposition of metal salts in aqueous medium or in a fluidised-bed reactor by gas-phase coating.

11. Use of the pearlescent pigments according to one of Claims 1 to 9 in paints, coatings, automobile paints, powder coatings, printing inks, security printing inks, plastics, ceramic materials, glasses, paper, in toners for electrophotographic printing processes, in seed, in greenhouse sheeting and tarpaulins, as absorbers in the laser marking of paper and plastics, as absorbers in the laser welding of plastics, in cosmetic formulations, for the preparation of pigment pastes with water, organic and/or aqueous solvents, for the preparation of pigment compositions and dry preparations.

12. Use of the pearlescent pigments according to one of Claims 1 to 9 for inhibiting the yellowing of polymers comprising phenolic additives.

13. Use of the pearlescent pigments according to Claim 11 in cosmetic formulations comprising dihydroxyacetone.

## Revendications

1. Pigments nacrés à base de paillettes de verre, **caractérisés en ce qu'**ils possèdent la structure en couches suivante :
(A) éventuellement une couche de SiO₂,
(B) un revêtement à indice de réfraction élevé ayant un indice de réfraction n > 1,8 constitué essentiellement de TiO₂,
(C) une couche à indice de réfraction faible comprenant du SiO₂ ou de l'Al₂O₃
et éventuellement
(D) une couche protectrice externe.

2. Pigments nacrés selon la revendication 1, **caractérisés en ce que** l'épaisseur de la couche (A) est de 2-350 nm.

3. Pigments nacrés selon la revendication 1 ou 2, **caractérisés en ce que** l'épaisseur de la couche (B) est de 20-300 nm.

4. Pigments nacrés selon l'une des revendications 1 à 3, **caractérisés en ce que** l'épaisseur de la couche (C) est de 2-200 nm.

5. Pigments nacrés selon l'une des revendications 1 à 4, **caractérisés en ce que** la couche (C) est en outre dopée par un ou plusieurs oxydes issus du groupe V, Zr, Zn, Ce, Ti, B, Na, K, Mg, Ca et/ou Mn.

6. Pigments nacrés selon l'une des revendications 1 à 5, **caractérisés en ce que** le TiO₂ dans la couche (B) se trouve selon la modification rutile.

7. Pigments nacrés selon la revendication 6, **caractérisés en ce que**, avant le revêtement par TiO₂, on applique une couche intermédiaire de SnO₂ ou de noyaux de SnO₂.

8. Pigments nacrés selon l'une des revendications 1 à 7, **caractérisés en ce qu'**ils possèdent la structure en couches suivante :
paillette de verre + SiO₂ + TiO₂ + SiO₂
paillette de verre + SiO₂ + TiO₂ + Al₂O₃
paillette de verre + TiO₂ + SiO₂
paillette de verre + TiO₂ + Al₂O₃.

9. Pigments nacrés selon l'une des revendications 1 à 8, **caractérisés en ce qu'**ils possèdent une couche protectrice externe (D) afin d'augmenter la stabilité à la lumière, à la température et aux intempéries.

10. Procédé de préparation des pigments nacrés selon l'une des revendications 1 à 9, **caractérisé en ce que** le revêtement des paillettes de verre est effectué par des méthodes chimiques par voie humide par la décomposition hydrolytique de sels métalliques en milieu aqueux ou dans un réacteur à lit fluidisé par revêtement en phase gazeuse.

11. Utilisation des pigments nacrés selon l'une des revendications 1 à 9 dans des peintures, des revêtements, des peintures pour automobiles, des revêtements pulvérulents, des encres d'impression, des encres d'impression de sécurité, des matières plastiques, des matériaux en céramique, des verres, du papier, dans des toners pour procédés d'impression électrophotographique, dans des semences, dans des toiles et des bâches pour serres, comme absorbants dans le marquage au laser de papier et de matières plastiques, comme absorbants dans le soudage au laser de matières plastiques, dans des formulations cosmétiques, pour la préparation de pâtes de pigments avec de l'eau, des solvants organiques et/ou aqueux, pour la préparation de compositions de pigments et de préparations sèches.

12. Utilisation des pigments nacrés selon l'une des revendications 1 à 9, pour l'inhibition du jaunissement des polymères comprenant des additifs phénoliques.

13. Utilisation des pigments nacrés selon la revendication 11, dans des formulations cosmétiques comprenant de la dihydroxyacétone.
